# EUROPÄISCHE PATENTSCHRIFT

(11) **EP 0 987 240 B1**
(45) Veröffentlichungstag und Bekanntmachung des Hinweises auf die Patenterteilung: **17.07.2002**
(21) Anmeldenummer: 99115269.5
(22) Anmeldetag: 31.07.1999
(51) Int. Cl.: C07C 29/141, C07C 31/125, C07C 29/16

(54) **Verfahren zur Hydrierung von Hydroformylierungsgemischen**
Process for hydrogenating hydroformylation mixtures
Procédé pour l'hydrogénation de mélanges d'hydroformylation

(30) Priorität: 16.09.1998 DE 19842369
(43) Veröffentlichungstag der Anmeldung: 22.03.2000
(73) Patentinhaber: Oxeno Olefinchemie GmbH, 45764 Marl (DE)
(72) Erfinder: Büschken, Wilfried, Dr., 45721 Haltern (DE); Gubisch, Dietmar, Dr., 45772 Marl (DE)

(56) Entgegenhaltungen:
- EP-A- 0 224 872
- DE-A- 1 935 900
- DE-C- 931 888
- FR-A- 2 322 119
- HOMER ADKINS ET AL: "The catalytic hydrogenation of organic compounds over copper chromite" JOURNAL OF THE AMERICAN CHEMICAL SOCIETY., 1931, Seiten 1091-1095, XP002123754 AMERICAN CHEMICAL SOCIETY, WASHINGTON, DC., US ISSN: 0002-7863

## Beschreibung

Die Erfindung betrifft ein Verfahren zur Hydrierung von Hydroformylierungsgemischen, die bei der Herstellung höherer Oxo-Alkohole durch Hydroformylierung der entsprechenden Olefine anfallen.

Höhere Alkohole, insbesondere solche mit 6 bis 25 C-Atomen, können bekanntlich durch katalytische Hydroformylierung (oder Oxo-Reaktion) der um ein C-Atom ärmeren Olefine und anschließende katalytische Hydrierung der Aldehyd- und Alkohol-haltigen Reaktionsgemische hergestellt werden. Sie werden vorwiegend als Edukte für die Herstellung von Weichmachern oder Detergentien verwendet.

Es ist bekannt, daß bei der katalytischen Hydroformylierung von Olefinen Reaktionsgemische entstehen, die außer den gewünschten Produkten, d.h. Aldehyden und den entsprechenden Alkoholen, in Abhängigkeft vom Katalysator und von den Reaktionsbedingungen Neben- und Folgeprodukte, wie nicht umgesetzte Olefine, durch Hydrierung aus den Olefinen entstandene gesättigte Kohlenwasserstoffe, Wasser. Ester der gewünschten Alkohole (z.B. Formiate), Acetale aus den Zielprodukten Aldehyd und Alkohol, Enolether sowie andere Neben- oder Folgeprodukte enthalten können. Man kann die genannten Stoffe in Leichtsieder mit einem Siedepunkt unterhalb des Siedepunktes des Aldehyds und Hochsieder mit einem Siedepunkt oberhalb des Siedepunktes des Alkohols unterteilen. Bei der Hydrierung der Reaktionsgemische entstehen aus manchen der Nebenprodukte, wie Estern und Acetalen, die als Zielprodukt gewünschten Alkohole, was die Ausbeute verbessert. Insbesondere ist es erwünscht, daß die Formiate, die in Mengen bis zu 10 Gew.-% vorkommen können, unter vergleichsweise milden Bedingungen und besonders unter niedrigem Druck mit handelsüblichen Katalysatoren zu dem gewünschten Alkohol (und zu Methanol als Nebenprodukt) hydriert werden.

Die katalytische Hydrierung von Reaktionsgemischen, die durch Kobaltkatalysierte Hydroformylierung von Olefinen mit 2 bis 24 C-Atomen hergestellt wurden, ist z.B. in DE 35 42 595 beschrieben. Die Hydrierung wird zweistufig durchgeführt. In der ersten Stufe wird das Hydroformylierungsgemisch bei 150-230°C und einem Wasserstoffdruck von 10-350 bar mit 80-95%igem Umsatz an einem SiO₂-Trägerkatalysator hydriert, der 5-15 Gew.-% Nickel und 2-20 Gew-% Molybdän in Form von Molybdänoxid enthält. In der zweiten Stufe wird die Hydrierung bei 150-230°C und 10-350 bar Wasserstoffdruck an einem Katalysator zu Ende geführt, dessen aktive Masse aus 55-60 Gew.-% Kobalt, 15-20 Gew.-% Kupfer, 4-10 Gew.-% Mangan und 2-5 Gew.-% Molybdän in Form von Molybdänoxid sowie gegebenenfalls bis zu 10 Gew.-% aktivierenden Zusätzen besteht. Bei dem Verfahren werden die im Gemisch vorliegenden Formiate und Acetale zu den entsprechenden Alkoholen umgesetzt. Das Verfahren hat jedoch den Nachteil, daß die Hydrierung in zwei Stufen und bei hohen Drücken - nach dem Beispiel bei 250 bzw. 245 bar - erfolgt.

In DE 19 35 900 ist ein Verfahren zur Hydrierung von Aldehyden und Ketonen aus der Oxo-Synthese beschrieben. Die Hydrierung wird an Cu/Cr-Katalysatoren in der Gasphase unter Anwesenheit von Kohlenmonoxid durchgeführt.

Nach US-A 5 399 793 werden für die Hydrierung von entkobalteten Reaktionsgemischen, wie sie bei der Hydroformylierung von C₅-C₁₂-Olefinen anfallen. Ni/Mo-Katalysatoren auf Al₂O₃ oder Al₂O₃·SiO₂ als Trägermaterialien verwendet. Das Gesamtverfahren umfaßt die folgenden Einzelschritte:
(a) Kobalt-katalysierte Hydroformylierung
(b) Entkobaltung des Reaktionsgemisches
(c) Hydrierung des rohen' Reaktionsgemisches bei erhöhter Temperatur und unter erhöhtem Druck
(d) Gewinnung von Alkoholen mit sehr geringen Mengen an Aldehyden durch Destillation und
(e) Finishhydrierung der Alkohole.

Die Hydrierungen der Stufen (c) und/oder (e) können unter Verwendung eines bimetallischen, Phosphor-freien Ni/Mo-Hydrierkatalysators durchgeführt werden. Dieser Hydrierkatalysator erzeugt weniger hochsiedende Nebenprodukte als ein entsprechender Phosphor-haltiger Katalysator. Nachteilig ist bei diesem Verfahren, daß zur Herstellung eines spezifikationsgerechten, für die Herstellung von Weichmachern geeigneten Alkohols zwei Hydrierstufen notwendig sind und daß zumindest bei der Hydrierstufe (b) ein relativ hoher Druck von 1.000 psig (etwa 70 bar) erforderlich ist.

Der vorliegenden Erfindung lag die Aufgabe zugrunde. Reaktionsgemische der Hydroformylierung von C₅- bis C₂₄-Olefinen unter vergleichsweise milden Bedingungen und insbesondere niedrigen Drücken an üblichen Katalysatoren mit hoher Standzeit so zu hydrieren, daß die Aldehyde und die als Nebenprodukte vorhandenen Formiate in die gewünschten Alkohole umgewandelt werden.

Diese Aufgabe wurde überraschend durch ein Verfahren zur Hydrierung von Reaktionsgemischen aus der Hydroformylierung von C₅- bis C₂₄-Olefinen mittels Wasserstoff an festen Katalysatoren bei erhöhter Temperatur gelöst, bei dem man aus dem Reaktionsgemisch die Aldehyde. Alkohole, Formiate und Leichtsieder verdampft, mittels eines Tröpfchenabscheiders mitgerissene Tröpfchen von Hochsiedern abscheidet und den Dampf über einen trägerfreien Cu/Cr-Katalysator leitet.

Das erfindungsgemäße Verfahren bietet eine Reihe von Vorteilen: Die im Verdampfer zurückbleibenden sowie die vorteilhaft aus den verdampften Aldehyden, Alkoholen, Formiaten und Leichtsiedern abgeschiedenen als Tröpfchen mitgerissenen Hochsieder werden nicht mithydriert. belasten also nicht die Hydrierstufe. Sie können, beispielsweise durch Spaltung (oder Cracken). zu Wertstoffen aufgearbeitet werden. Die Aldehyde werden bei Umsätzen über 98% in einer Selektivität von über 99% in nur einer Hydrierstufe zu den entsprechenden Alkoholen hydriert. Die enthaltenen Ester, insbesondere die Formiate. werden ebenfalls zu den erwünschten Alkoholen hydriert. Die Hydrierung kann im Niederdruckbereich von unter 25 bar durchgeführt werden. Ein erwünschter Nebeneffekt besteht darin, daß die im Reaktionsgemisch enthaltenen Ausgangsolefine überwiegend nicht hydriert werden. was ihre Rückführung in die Hydroformylierungsreaktion ermöglicht.

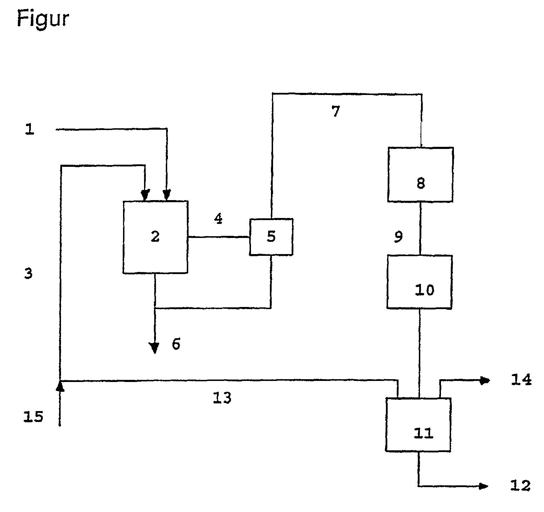

In der Figur ist das Blockschema einer Anlage dargestellt, in der das erfindungsgemäße Verfahren mit Kreisgasführung des Hydrierwasserstoffs kontinuierlich durchgeführt werden kann. Das Hydroformylierungsgemisch wird als Edukt 1 in den Verdampfer 2 eingeführt, der im Gleichstrom von erhitztem Wasserstoff 3 durchströmt wird. Der mit Aldehyden. Alkoholen. Formiaten und Leichtsiedern beladene Wasserstoffstrom 4 wird durch den Tröpfchenabscheider 5 geführt, die dert abgeschiedenen und die im Verdampfer 2 zurückbleibenden Hochsieser werden als Hochsiederanteil 6 kontinuierlich oder absatzweise abgezogen. Der von Hochsiedern befreite, mit Aldehyden, Alkohole, Formiaten und Leichtsiedern beladene Wasserstoffstrom 7 wird in den Hydrierreaktor 8 geleitet, aus dem das Hydrierungsgemisch 9 austritt, das im Kühler 10 gekühlt wird. In der Produktvorlage 11 trennt sich das Hydrierungsgemisch in Hydrierungsprodukt 12 und Kreisgas 13, von dem ein Teil als Abgas 14 abgezogen wird, um den Inertgasspiegel auf akzeptabler Höhe zu halten. Der verbrauchte Wasserstoff wird durch Frischwasserstoff 15 ersetzt.

Die Edukte für die Hydroformylierung sind Monoolefine mit 5 bis 24 Kohlenstoffatomen und end- oder mittelständiger C-C-Doppelbindung oder Gemische solcher Monoolefine, wie 1- oder 2-Penten, 2-Methyl-1-buten, 1-, 2- oder 3-Hexen, das bei der Dimerisierung von Propen anfallende isomere C₆-Olefingemisch (Dipropen), 3-Methyl-1-hexen, 1-Octen, das bei der Dimerisierung von Butenen anfallende isomere C₈-Olefingemisch (Dibuten), 1-Nonen, 2-, 3- oder 4-Methyl-1-octen, das bei der Trimerisierung von Propen anfallende isomere C₉-Olefingemisch (Tripropen), 1-, 2- oder 3-Decen, 2-Ethyl-1-octen, 1-Dodecen, das bei der Tetramerisierung von Propen oder der Trimerisierung von Butenen anfallende isomere C₁₂-Olefingemisch (Tetrapropen oder Tributen), 1-Tetradecen, 1- oder 2-Hexadecen, bei der Tetramerisierung von Butenen anfallende C₁₆-Olefingemische (Tetrabuten) sowie durch Cooligomerisierung von Olefinen mit unterschiedlichen C-Zahlen (bevorzugt 2 bis 4) hergestellte Olefingemische, gegebenenfalls nach destillativer Trennung in Fraktionen mit gleicher oder ähnlicher C-Zahl. Vorzugsweise werden Gemische hydriert, die bei der Hydroformylierung von C₈-, C₉-, C₁₂- oder C₁₆-Olefingemischen entstehen.

Die Olefine werden in üblicher Weise hydroformyliert und ergeben dann die Edukte für das erfindungsgemäße Hydrierverfahren. Man arbeitet also mit Rhodium- oder vorzugsweise mit Kobaltkatalysatoren sowie mit oder ohne Komplex-stabilisierende Zusätze, wie organische Phosphine oder Phosphite. Die Temperaturen und die Drücke können, je nach Katalysator und Olefin, in weiten Grenzen variieren. Eine Beschreibung der Hydroformylierung von Olefinen findet sich z.B. bei J.Falbe. New Syntheses with Carbon Monoxide, Springer-Verlag, Heidelberg-New York. 1980, Seiten 99ff, sowie bei Kirk-Othmer, Encyclopedia of Chemical Technology, Band 17, 4. Auflage, John Wiley & Sons, Seiten 902-919 (1996).

Die Reaktionsgemische der Hydroformylierung werden zweckmäßig zunächst vom Katalysator befreit. Wenn ein Kobaltkatalysator verwendet wurde, kann dies durch Druckentlastung, Abtrennung der wäßrigen Katalysatorphase. Oxidation der im Hydroformylierungsgemisch verbliebenen Kobaltcarbonylverbindungen mit Luft oder Sauerstoff und Auswaschen der entstandenen Kobaltverbindungen mit Wasser oder wäßriger Säure geschehen. Entkobaltungsverfahren sind gut bekannt, siehe z.B. J.Falbe, a.a.0., 164, 165 (BASF-Process); Kirk-Otmer, a.a.0., sowie EP-0 850 905 Al.

Wenn eine Rhodiumverbindung als Hydroformylierungskatalysator diente, kann man sie mittels Dünnschichtverdampfung als Destillationsrückstand vom Hydroformylierungsgemisch abtrennen.

Die zweckmäßig von Katalysator befreiten Reaktionsgemische der Hydroformylierung enthalten im allgemeinen 3-40 Gew.-%, meistens 5-30 Gew.-% Leichtsieder, weiterhin 30-90 Gew.-% Aldehyde, 5-60 Gew.-% Alkohole, bis zu 10-% Formiate dieser Alkohole und 5 bis 15 Gew.-% Hochsieder. Es sei jedoch betont, daß das erfindungsgemäße Verfahren auch mit Hydroformylierungsgemischen ausgeführt werden kann, deren Zusammensetzung in dieser und/oder jener Beziehung nicht diesen Angaben entspricht.

Aus dem gegebenenfalls von Katalysator befreiten, in der Regel undestillierten Reaktionsgemisch der Hydroformylierung werden zunächst die Aldehyde, Alkohole, Formiate und Leichtsieder verdampft. Zu den Leichtsiedern zählen' vor allem nicht umgesetzte Olefine und die während der Hydroformylierung entstandenen 'entsprechenden gesättigten Kohlenwasserstoffe sowie Wasser. Der Anteil der Leichtsieder am Reaktionsgemisch schwankt je nach dem Ausgangsolefin, den Reaktionsbedingungen und dem Umsatzgrad der Hydroformylierung in den zuvor genannten Grenzen. Die Hochsieder, die erfindungsgemäß nicht zum Hydrierkatalysator gelangen sollen, enthalten u.a. Aldolisierungs und/oder Kondensationsprodukte der entstandenen Aldehyde sowie Acetale und Enolether und sieden als höhermolekulare Stoffe erheblich höher als die Alkohole, Aldehyde, Formiate und Leichtsieder.

Die Bedingungen, unter denen Aldehyde, Alkohole, Formiate und Leichtsieder von den Hochsiedern getrennt werden, hängen erheblich von der C-Zahl der Ausgangsolefine ab. Die Trennung des Reaktionsgemisches erfolgt zweckmäßig unter denselben Bedingungen hinsichtlich Temperatur und Druck, unter denen die nachfolgende Hydrierung durchgeführt wird. Der Druck liegt dementsprechend in der Regel unterhalb von 25 bar. Er beträgt zweckmäßig 1 bis 25 bar und insbesondere 15 bis 20 bar. Im Fall von Reaktionsgemischen aus der Hydroformylierung von Olefinen mit 6 bis 12 C-Atomen (beispielsweise von Octenen, die durch Dimerisierung von Butenen erhalten wurden) kann man z.B. bei Temperaturen von 150 bis 250°C, vorteilhaft von 160 bis 220°C arbeiten. Für andere Hydroformylierungsgemische lassen sich die optimalen Temperaturbedingungen für die Abtrennung der Schwersieder durch orientierende Versuche unschwer ermitteln.

Zur Auftrennung der Hydroformylierungsgemische verwendet man übliche Vorrichtungen, z.B. Dünnschicht- oder Fallfilmverdampfer. Bei einer vorteilhaften Ausführungsform dosiert man das Gemisch in einen Wasserstoffstrom mit entsprechender Temperatur. Unabhängig von der gewählten Verdampfungsmethode ist es vorteilhaft, den Dampfstrom von Schwersiedertröpfchen zu befreien, da auf diese Weise die Standzeit des Katalysators erhöht wird. Man verwendet übliche Tröpfchenabscheider, in denen die Geschwindigkeit des Dampfstromes vermindert wird. der Dampfstrom der Einwirkung von Zentrifugalkräften ausgesetzt wird oder die Tröpfchen durch Aufprall, z.B. auf Prallbleche oder Siebe, abgeschieden werden.

Wenn das Hydroformylierungsgemisch durch Einbringen in einen erhitzten Wasserstoffstrom verdampft wurde, führt man das Wasserstoff/-Dampfgemisch über den Katalysator. Anderenfalls mischt man dem Dampfgemisch Wasserstoff zu. Der Wasserstoff wird zweckmäßig in einem erheblichen stöchiometrischen Überschuß angewandt. Vorteilhaft arbeitet man mit einem Wasserstoff:Edukt-Massenverhältnis von 3,5:1 bis 0,7:1, insbesondere von 3:1 bis 1.1. Der nicht verbrauchte Wasserstoff kann im Kreis geführt werden.

Der Katalysator ist ein trägerfreier Cu/Cr-Katalysator. Er wird zweckmäßig als Festbettkatalysator angewandt und enthält im allgemeinen 25 bis 40 Gew.-% Kupfer und 18 bis 30 Gew.-% Chrom. Der Katalysator kann bis zu 20 Gew.-% an basischen Stoffen, wie Akali- oder Erdalkalioxiden oder -hydroxiden, sowie andere, inerte oder eigenschaftsmodifizierende Stoffe in denselben Mengen enthalten, beispielsweise Graphit. "Trägerfrei" bedeutet, daß kein Trägermaterial vorhanden ist, das mit einer Lösung der aktiven Komponenten besprüht oder getränkt wurde oder auf das die aktiven Komponenten auf andere Weise haftend aufgebracht wurden. Der zunächst oxidische Katalysator wird zweckmäßig bei erhöhter Temperatur, z.B. der Hydriertemperatur. durch Überleiten von Wasserstoff reduziert und entfaltet dann seine optimale Wirksamkeit. Die angegebenen Gewichtsanteile beziehen sich auf die oxidische, nicht reduzierte Form des Katalysators. Geeignete Katalysatoren sind z.B. der Katalysator E406TU von Mallinckrodt. Erie, Pennsylvania, USA sowie der Katalysator G99B der Süd-Chemie AS. 80333 München. Die Katalysatoren werden zweckmäßig in einer Form eingesetzt, die einen geringen Strömungswiderstand bietet, z.B. in Form von Granalien, Pellets oder Formkörpern, wie Tabletten, Zylindern. Strangextrudaten oder Ringen. Die Temperaturen am Katalysator sowie der Druck entsprechen, wie erwähnt, vorteilhaft den Bedingungen, unter denen die Aldehyde, Alkohole, Formiate und Leichtsieder aus dem Hydroformylierungsgemisch verdampft werden.

Die optimale Temperatur im Katalysatorbett wird für ein gegebenes Hydroformylierungsgemisch zweckmäßig durch orientierende Versuche ermittelt. Bei Hydroformylierungsgemischen, die durch Hydroformylierung von Olefinen mit 6 bis 12 C-Atomen erhalten wurden, liegt sie, wie erwähnt, bei 150 bis 250°C, vorteilhaft bei 160 bis 220°C. Es ist zweckmäßig, daß die Temperatur des Kreisgases mit dem verdampften Teil des Hydroformylierungsgemisches am Eingang des Hydrierungsreaktors mindestens so hoch ist wie am Ausgang des Tröpfchenabscheiders. Vorteilhaft wird dementsprechend vorgesehen, daß das Kreisgas in diesem Verfahrensabschnitt beheizt, zweckmäßig indirekt beheizt, und thermostatisiert werden kann.

Die Hydrierung verläuft exotherm. Man kann die Reaktion unter Temperaturanstieg adiabatisch führen. Alternativ ist es auch möglich, die Hydrierung im wesentlichen isotherm zu gestalten, d.h. einen Temperaturanstieg vom Eingang des Reaktors bis zum Austritt des Hydrierungsgemisches von nur bis zu 10°C zuzulassen. Im letzteren Falle regelt man die Temperatur zweckmäßig durch Einspeisung von kaltem Hydrierwasserstoff.

Das erfindungsgemäße Verfahren erlaubt hohe Durchsätze. Die Katalysatorbelastung (liquid hourly space velocity = LHSV) wird als stündlicher Volumenstrom des noch flüssigen Edukts dividiert durch das Katalysatorvolumen angegeben. Sie hängt u.a. von der gewählten Temperatur ab und liegt im allgemeinen zwischen 0,07h⁻¹ und 0,40h⁻¹, insbesondere zwischen 0.12h⁻¹ und 0.25h⁻¹. Die Verweilzeit der Gasphase in der Katalysatorzone hängt im wesentlichen von der GHSV (gas hourly space velocity), der gewählten Temperatur und dem Druck ab und kann z.3. zwischen 3 und 30 sec liegen.

Das Hydrierungsgemisch kann nach Abtrennung von überschüssigem Wasserstoff durch fraktionierte Kondensation oder durch vollständige Kondensation und Destillation in seine Bestandteile zerlegt werden. Aus dem Leichtsiederanteil können die nicht hydrierten Olefine zurückgewonnen werden, vorteilhaft durch Destillation, und in die Hydroformylierung zurückgeführt werden. Alternativ kann man die Olefine zusammen mit den aus ihnen bei der Hydroformylierung oder der Hydrierung entstandenen gesättigten Kohlenwasserstoffen als Rohstoff für Cracker oder zu Heizzwecken verwenden. Das gilt besonders dann, wenn die Hydroformylierung mit hohem Umsatz der Ausgangsolefine gefahren wurde. Die Alkohole fallen in einer durch gaschromatographische Analyse bestimmten Reinheit von >99% an. Der Rückstand kann mit den Hochsiedern, die bei der Verdampfung der Aldehyde, Alkohole, Formiate und Leichtsieder zurückgeblieben waren, vereinigt und zusammen mit ihnen auf Wertstoffe aufgearbeitet werden. Beispielsweise kann man durch Cracken Olefine gewinnen, die wiederum hydroformyliert werden können.

Die folgenden Beispiele sollen die Erfindung weiter erläutern, nicht aber ihren Anwendungsbereich beschränken, der sich aus den Patentansprüchen ergibt.

### Beispiel 1

In den Verdampfer 2 einer Gasphasen-Hydrierapparatur gemäß der Figur wurde mit einer Dosierpumpe als Edukt 1 hydroformyliertes Di-n-buten in einen auf 200°C erhitzen Wasserstoffstrom 3 eingespeist. Der den Verdampfer verlassende, mit Aldehyden, Alkoholen, Formiaten und Leichtsiedern beladene und Hochsiedertröpfchen enthaltende Wasserstoffstrom 4 wurde über den Tröpfchenabscheider 5 geleitet und nach Abtrennung von Hochsiedern als Stoffstrom 7 auf den Kopf des Hydrierreaktors 8 geleitet. Die im Verdampfer zurückbleibenden und im Tröpfchenabscheider 5 abgeschiedenen Hochsieder 6 wurden alle 12 h abgezogen.

Der Reaktor war ein Stahlrohr von 38 mm lichter Weite, in dem 800 ml (=1200 g) des Katalysators E406TU von Mallinckrodt in Form von Pellets fest angeordnet war. Der Katalysator enthielt in seiner oxidischen, unreduzierten Form

| | |
|---|---|
| 42 Gew.-% | CuO, entsprechend 33.55 Gew.-% Cu |
| 40 Gew.-% | Cr₂O₃, entsprechend 27.37 Gew.-% Cr |
| 8 Gew.-% | BaO und |
| 10 Gew.-% | Graphit. |

Der Katalysator wurde reduziert, indem bei 150°C und 1 bar über 800 ml Katalysator zunächst 2500 Nl/h Stickstoff geleitet wurden. Maximal 5% des Stickstoffs wurden durch Wasserstoff ersetzt und der Gasstrom so geregelt, daß der Temperaturanstieg unter 10°C blieb. Nach jeweils 2 h wurde die Volumenkonzentration des Wasserstoffs um 5 % angehoben. Nachdem schließlich mit reinem Wasserstoff reduziert worden war, wurde die Temperatur auf 160°C erhöht. Nach jeweils weiteren 2 h wurde die Temperatur um jeweils 10°C gesteigert. Nachdem eine Temperatur von 190°C erreicht war, wurde der Wasserstoffdruck unter strenger Temperaturkontrolle stufenweise auf 15 bar erhöht und der Katalysator 12 h unter diesen Bedingungen gehalten.

Das Hydrierungsgemisch wurde im Kühler 10 gekühlt. Aus der Vorlage 11 wurde das kondensierte Hydrierungsprodukt 12 abgezogen, das Kreisgas 13 ging in den Verdampfer zurück, und ein Teil des Kreisgases wurde als Abgas 14 abgezogen. Der verbrauchte Wasserstoff wurde durch Frischwasserstoff 15 ersetzt.

Das Verfahren wurde unter den folgenden Bedingungen durchgeführt:

| | |
|---|---|
| Zugeführtes Edukt | 160 g/h |
| Zugeführter Frischwasserstoff | 62 Nl/h |
| Kreisgaswasserstoff | 2500 Nl/h |
| Abgas | 50 Nl/h |
| Hochsieder | 3 g/h |
| Temperatur nach dem Verdampfer | 180 °C |
| Temperatur am Hydrierreaktoreingang | 185 °C |
| Temperatur im Hydrierreaktor | 185 °C |
| Druck im System | 16 bar |
| Hydrierungsprodukt | ca. 158 g/h |

GC-Analysen des Edukts sowie des Hydrierungsprodukts ergaben folgende Werte:

| Stoff | Edukt (Gew.-%) | Produkt (Gew.-%) |
|---|---|---|
| Isononanale | 39,4 | 0,2 |
| Isononanole | 41,8 | 87,9 |
| Isononylformiate | 4,2 | < 0.1 |
| Hochsieder | 5,9 | 2.2 |

Nach einer Woche Betrieb war ein stationärer Zustand erreicht, und die Produktzusammensetzung blieb für einen Zeitraum von mehr als 6 Monaten gleich.

### Beispiel 2

Wurde der gleiche Versuch ohne Tröpfchenabscheider unter sonst gleichen Bedingungen durchgeführt, so war schon nach 6 Wochen eine deutlich verschlechterte Hydrierleistung festzustellen, erkennbar an einem höheren Gehalt an Isononylformiaten.

## Patentansprüche

1. Verfahren zur Hydrierung von Reaktionsgemischen aus der Hydroformylierung von C₅bis C₂₄-Olefinen mittels Wasserstoff an festen Katalysatoren bei erhöhter Temperatur,
**dadurch gekennzeichnet,**
**dass** man aus dem Reaktionsgemisch die Aldehyde, Alkohole, Formiate und Leichtsieder verdampft, mittels eines Tröpfchenabscheiders mitgerissene Tröpfchen von Hochsiedern abscheidet und den Dampf über einen trägerfreien Cu/Cr-Katalysator leitet.

2. Verfahren nach Anspruch 1,
**dadurch gekennzeichnet,**
**dass** man das Verfahren kontinuierlich durchführt.

3. Verfahren nach Anspruch 1 oder 2,
**dadurch gekennzeichnet,**
**dass** man die im Tröpfchenabscheider und im Verdampfer anfallenden Hochsieder auf Wertstoffe aufarbeitet.

4. Verfahren nach einem der Ansprüche 1 bis 3,
**dadurch gekennzeichnet,**
**dass** das Kreisgas nach Verlassen des Tröpfchenabscheiders und vor dem Eintritt in den Hydrierreaktor beheizt und thermostatisiert werden kann.

5. Verfahren nach einem der Ansprüche 1 bis 4,
**dadurch gekennzeichnet,**
**dass** die Temperatur des Kreisgases am Eingang des Hydrierreaktors mindestens so hoch wie am Ausgang des Tröpfchenabscheiders ist.

6. Verfahren nach einem der Ansprüche 1 bis 5,
**dadurch gekennzeichnet,**
**dass** der Katalysator 25 bis 40 Gew.-% Cu und 18 bis 30 Gew.-% Cr, bezogen auf die oxidische Form des Katalysators, enthält.

7. Verfahren nach Anspruch 6,
**dadurch gekennzeichnet,**
**dass** der Katalysator bis zu 20 Gew.-% eines basischen Stoffs enthält.

8. Verfahren nach Anspruch 7,
**dadurch gekennzeichnet,**
**dass** der Katalysator bis zu 20 Gew.-% eines inerten oder eigenschaftsmodifizierten Stoffs enthält.

9. Verfahren nach einem der Ansprüche 1 bis 8,
**dadurch gekennzeichnet,**
**dass** man die Hydrierung an einem Festbettkatalysator durchführt.

10. Verfahren nach einem der Ansprüche 1 bis 9,
**dadurch gekennzeichnet,**
**dass** man die Hydrierung bei 150 bis 250 °C durchführt.

11. Verfahren nach einem der Ansprüche 1 bis 10,
**dadurch gekennzeichnet,**
**dass** man die Hydrierung bei 160 bis 220 °C durchführt.

12. Verfahren nach einem der Ansprüche 1 bis 11,
**dadurch gekennzeichnet,**
**dass** man die Hydrierung adiabatisch durchführt.

13. Verfahren nach einem der Ansprüche 1 bis 12,
**dadurch gekennzeichnet,**
**dass** man die Hydrierung im Wesentlichen isotherm durchführt, indem man die Temperatur durch Einspeisung von Kaltgas regelt.

14. Verfahren nach einem der Ansprüche 1 bis 13,
**dadurch gekennzeichnet,**
**dass** man die Hydrierung bei einem Druck von 1 bis 25 bar durchführt.

15. Verfahren nach einem der Ansprüche 1 bis 14,
**dadurch gekennzeichnet,**
**dass** man die Hydrierung bei einem Druck von 1 bis 20 bar durchführt.

16. Verfahren nach einem der Ansprüche 1 bis 15,
**dadurch gekennzeichnet,**
**dass** die Katalysatorbelastung zwischen 0,07h⁻¹ und 0,40h⁻¹ liegt.

17. Verfahren nach einem der Ansprüche 1 bis 16,
**dadurch gekennzeichnet,**
**dass** die Katalysatorbelastung zwischen 0,12h⁻¹ und 0,25h⁻¹ liegt.

18. Verfahren nach einem der Ansprüche 1 bis 17,
**dadurch gekennzeichnet,**
**dass** man die Hydrierung mit einem Wasserstoff:Edukt-Massen-Verhältnis von 3,5:1 bis 0,7:1 durchführt.

19. Verfahren nach einem der Ansprüche 1 bis 17,
**dadurch gekennzeichnet,**
**dass** man die Hydrierung mit einem Wasserstoff:Edukt-Massen-Verhältnis von 3:1 bis 1:1 durchführt.

20. Verfahren nach einem der Ansprüche 1 bis 19,
**dadurch gekennzeichnet,**
**dass** man als Edukt Hydroformylierungsgemische einsetzt, die durch mittels Kobaltkatalysierter Hydroformylierung hergestellt wurden.

21. Verfahren nach Anspruch 20,
**dadurch gekennzeichnet,**
**dass** man die Hydroformulierungsgemische undestilliert verwendet.

## Claims

1. A process for the hydrogenation of reaction mixtures from the hydroformylation of C₅ to C₂₄ olefins using hydrogen on fixed catalysts at elevated temperature **characterized in that** the aldehydes, alcohols, formates and low-boilers are evaporated from the reaction mixture, entrained droplets of high-boilers are separated off using a demister, and the vapor is passed over a support-free Cu/Cr catalyst.

2. A process according to claim 1, **characterized in that** the process is carried out continuously.

3. A process according to claim 1 or 2, **characterized in that** the high-boilers produced in the demister and in the evaporator are worked up to materials of value.

4. A process according to any one of claims 1 to 3, **characterized in that** the cycle gas, after leaving the demister and upstream of entry into the hydrogenation reactor, can be heated and thermostated.

5. A process according to any one of claims 1 to 4, **characterized in that** the temperature of the cycle gas at the inlet of the hydrogenation reactor is at least as high as at the outlet of the demister.

6. A process according to any one of claims 1 to 5, **characterized in that** the catalyst comprises from 25 to 40% by weight of Cu and from 18 to 30% by weight of Cr, based on the oxidic form of the catalyst.

7. A process according to claim 6, **characterized in that** the catalyst comprises up to 20% by weight of a basic substance.

8. A process according to claim 7, **characterized in that** the catalyst comprises up to 20% by weight of an inert or property-modifying substance.

9. A process according to any one of claims 1 to 8, **characterized in that** the hydrogenation is carried out on a fixed-bed catalyst.

10. A process according to any one of claims 1 to 9, **characterized in that** the hydrogenation is carried out at from 150 to 250°C.

11. A process according to any one of claims 1 to 10, **characterized in that** the hydrogenation is carried out from 160 to 220°C.

12. A process according to any one of claims 1 to 11, **characterized in that** the hydrogenation is carried out adiabatically.

13. A process according to any one of claims 1 to 12, **characterized in that** the hydrogenation is essentially carried out isothermically, by controlling the temperature by feeding cold gas.

14. A process according to any one of claims 1 to 13, **characterized in that** the hydrogenation is carried out at a pressure of from 1 to 25 bar.

15. A process according to any one of claims 1 to 14, **characterized in that** the hydrogenation is carried out at a pressure of from 1 to 20 bar.

16. A process according to any one of claims 1 to 15, **characterized in that** the liquid hourly space velocity of the catalyst is between 0.07 h⁻¹ and 0.40 h⁻¹.

17. A process according to any one of claims 1 to 16, **characterized in that** the liquid hourly space velocity of the catalyst is between 0.12 h⁻¹ and 0.25 h⁻¹.

18. A process according to any one of claims 1 to 17, **characterized in that** the hydrogenation is carried out at a hydrogen:starting material mass ratio of from 3.5:1 to 0.7:1.

19. A process according to any one of claims 1 to 17, **characterized in that** the hydrogenation is carried out at a hydrogen:starting material mass ratio of from 3:1 to 1:1.

20. A process according to any one of claims 1 to 19, **characterized in that**, as starting material, use is made of hydroformylation mixtures which have been prepared by cobalt-catalyst hydroformylation.

21. A process according to claim 20, **characterized in that** the hydroformylation mixtures are used undistilled.

## Revendications

1. Procédé d'hydrogénation de mélanges réactionnels provenant de l'hydroformylation d'oléfines en C₅ à C₂₄, à l'aide d'hydrogène sur des catalyseurs solides à température accrue,
**caractérisé en ce qu'**
on évapore du mélange réactionnel les aldéhydes, les alcools, les formiates et les produits à bas point d'ébullition, on sépare à l'aide d'un collecteur de gouttelettes, les gouttelettes entraînées de produits à point. d'ébullition élevé et on conduit la vapeur sur un catalyseur Cu/Cr dépourvu de support.

2. Procédé selon la revendication 1,
**caractérisé en ce qu'**
on exécute le procédé en continu.

3. Procédé selon la revendication 1 ou la revendication 2,
**caractérisé en ce qu'**
on traite les produits à point d'ébullition élevé qui se produisent dans le collecteur de gouttelettes et dans l'évaporateur pour les substances de valeur.

4. Procédé selon l'une quelconque des revendications 1 à 3,
**caractérisé en ce que**
le gaz du circuit après départ du collecteur de gouttelettes et avant l'entrée dans le réacteur d'hydrogénation, peut être chauffé et thermostaté.

5. Procédé selon l'une quelconque des revendications 1 à 4,
**caractérisé en ce que**
la température du gaz de circuit est à l'entrée du réacteur d'hydrogénation au moins aussi élevée qu'à la sortie du collecteur de gouttelettes.

6. Procédé selon l'une quelconque des revendications 1 à 5.
**caractérisé en ce que**
le catalyseur contient de 25 à 40 % en poids de Cu et de 18 à 30 % de Cr rapporté à la forme oxydique du catalyseur.

7. Procédé selon la revendication 6,
**caractérisé en ce que**
le catalyseur contient jusqu'à 20 % en poids de substance basique.

8. Procédé selon la revendication 7,
**caractérisé en ce que**
le catalyseur contient jusqu'à 20 % d'une substance inerte ou qui modifie les propriétés.

9. Procédé selon l'une quelconque des revendications 1 à 8,
**caractérisé en ce qu'**
on exécute l'hydrogénation sur un catalyseur sur lit fixe.

10. Procédé selon l'une quelconques des revendications 1 à 9,
**caractérisé en ce qu'**
on exécute l'hydrogénation à 150 à 250°C.

11. Procédé selon l'une quelconque des revendications 1 à 10,
**caractérisé en ce qu'**
on exécute l'hydrogénation à 160 à 220°C.

12. Procédé selon l'une quelconque des revendications 1 à 11,
**caractérisé en ce qu'**
on exécute l'hydrogénation d'une manière adiabatique.

13. Procédé selon l'une quelconque des revendications 1 à 12,
**caractérisé en ce qu'**
on exécute l'hydrogénation pour l'essentiel d'une manière isotherme, en réglant la température par injection de gaz froid.

14. Procédé selon l'une quelconque des revendications 1 à 13,
**caractérisé en ce qu'**
on exécute l'hydrogénation à une pression allant de 1 à 25 bars.

15. Procédé selon l'une quelconque des revendications 1 à 14,
**caractérisé en ce que**
on exécute l'hydrogénation à une pression allant de 1 à 20 bars.

16. Procédé selon l'une quelconque des revendications 1 à 15,
**caractérisé en ce que**
la charge en catalyseur se situe entre 0,07h⁻¹ et 0.40h⁻¹.

17. Procédé selon l'une quelconque des revendications 1 à 16,
**caractérisé en ce que**
la charge en catalyseur se situe entre 0,12h⁻¹ t 0,25h¹.

18. Procédé selon l'une quelconque des revendications 1 à 17,
**caractérisé en ce qu'**
on exécute l'hydrogénation avec un rapport hydrogène/éduit/produit de 3,5 : 1 à 0,7 : 1.

19. Procédé selon l'une quelconque des revendications 1 à 17,
**caractérisé en ce qu'**
on exécute l'hydrogénation avec un rapport hydrogène : masses d'éduit allant de 3 : 1, à 1 : 1.

20. Procédé selon l'une quelconque des revendications 1 à 19,
**caractérisé en ce que**
comme éduits, on met en oeuvre des mélanges d'hydroformylation produits par hydroformylation catalysée par du cobalt.

21. Procédé selon la revendication 20,
**caractérisé en ce qu'**
on utilise les mélanges d'hydroformylation non distillés.
